# DEMANDE DE BREVET EUROPEEN

(11) **EP 1 350 463 A1**
(43) Date de publication de la demande: **08.10.2003**
(21) Numéro de dépôt: 03290664.6
(22) Date de dépôt: 17.03.2003
(51) Int. Cl.: A61B 5/103

(54) **Dispositif pour mesurer les caractéristiques de tissus**

(30) Priorité: 18.03.2002 FR 0203322
(71) Demandeur: Gharbi, Tijani, 25000 Besancon (FR); Armbruster, Vincent Thomas, 70100 Gray (FR); Humbert, Philippe Gérard Lucien, 25290 Ornans (FR)
(72) Inventeur: Gharbi, Tijani, 25000 Besancon (FR); Armbruster, Vincent Thomas, 70100 Gray (FR); Humbert, Philippe Gérard Lucien, 25290 Ornans (FR)
(74) Mandataire: Garel, Régis

(57) **Abrégé**

Dispositif de mesure d'une caractéristique physique d'un tissu biologique (2), comportant un palpeur (3) muni d'un élément de contact (7) présentant une surface de contact (8) destinée à venir en appui sur ledit tissu biologique, des moyens de sollicitation mécanique dudit palpeur et des moyens de mesures des déplacements ou des efforts résultant des sollicitations mécaniques.

Les moyens de sollicitation mécanique sont adaptés pour solliciter le palpeur (3) dans un plan parallèle à la surface de contact (8) de l'élément de contact, l'élément de contact (7) est réalisé à partir d'une matière rigide et la surface de contact (8) dudit élément de contact présente une pluralité de micro-reliefs saillants destinés à venir au contact du tissu.

## Description

La présente invention se rapporte à un dispositif de mesure d'au moins une caractéristique physique d'un tissu biologique, comportant un palpeur muni d'un élément de contact présentant une surface de contact destinée à venir en appui sur ledit tissu biologique, des moyens de sollicitation mécanique dudit palpeur et des moyens de mesures des déplacements ou des efforts résultant des sollicitations mécaniques.

Pour mesurer certaines caractéristiques physiques de tissus biologiques tels que notamment la peau, on utilise généralement un dispositif de mesure comportant un palpeur faisant office d'élément de contact et qui vient en appui sur le tissu à étudier. Par exemple, pour déterminer des caractéristiques mécaniques de la peau, le dispositif de mesure comprend, d'une part, des moyens pour appliquer sur la peau une sollicitation, telle qu'une force ou un déplacement, et d'autre part, des moyens d'enregistrement des déplacements ou des efforts résultants de cette sollicitation. Le traitement des données ainsi obtenues permet de déterminer une caractéristique physique du tissu biologique, soit dans un but de diagnostic médical, soit pour évaluer les effets de produits cosmétiques appliqués préalablement sur la peau d'un patient.

Par exemple, le document LINDHAL O A et al : *"Impression technique for the assessment of oedema: comparison with a new tactile sensor that measures physical properties of tissue"* Medical and Biological Engineering and Computing, Peter PEREGRINUS Ltd. STEVENAGE, GB, vol. 33, No. 1, 1995, pages 27-32, X000486782, décrit, entre autres, un dispositif de mesure comportant un palpeur qui exerce une pression sur un tissu vivant de manière perpendiculaire à celui-ci afin de diagnostiquer différents types d'oedèmes.

Toutefois, l'évaluation de certaines caractéristiques des couches superficielles du tissu biologique, comme par exemple l'élasticité de la peau, impose d'exercer des contraintes de cisaillements à la surface du tissu. Dans ce cas, le dispositif ou plus exactement l'élément de contact, exerce sur le tissu une sollicitation dans un plan tangentiel à celui-ci, puis le dispositif mesure l'effet produit par cette sollicitation. La surface de contact de l'élément de contact formé par le palpeur doit alors accrocher correctement au tissu afin que ledit élément ne glisse pas par rapport à celui-ci, car tout glissement entraînerait une erreur de mesure.

Afin de pallier le risque de glissement, il est possible d'augmenter la force d'appui du palpeur sur le tissu ou de diminuer la surface de contact dudit palpeur avec le tissu en utilisant un palpeur pointu. Mais ces solutions présentent des limites liées à la résistance du tissu biologique ou à l'apparition de douleurs s'il s'agit de la peau d'un être humain.

La présente invention a pour but de pallier les problèmes techniques mentionnés ci-dessus, en proposant un dispositif de mesure adapté notamment pour évaluer l'élasticité des couches superficielles d'un tissu biologique et muni d'un élément de contact présentant un bon accrochage avec un tissu comme la peau, sans toutefois la blesser, et pouvant facilement être mis en oeuvre à un coût acceptable.

A cet effet, l'invention a pour objet un dispositif de mesure du type précité caractérisé en ce que les moyens de sollicitation mécanique sont adaptés pour solliciter le palpeur dans un plan parallèle à la surface de contact de l'élément de contact, et en ce que l'élément de contact est réalisé à partir d'une matière rigide et la surface de contact dudit élément de contact présente une pluralité de micro-reliefs saillants destinés à venir au contact du tissu.

Dans des formes de réalisations préférées de l'invention, on a recours, en outre, à l'une et ou à l'autre des dispositions suivantes :
- la hauteur des micro-reliefs de la surface de contact est inférieure à 1 millimètre ;
- les micro-reliefs sont uniformément répartis sur toute la surface de contact ;
- les micro-reliefs de la surface de contact sont obtenus par usinage chimique ;
- la matière rigide de l'élément de contact est réalisée à partir de silicium ;
- l'usinage chimique de l'élément de contact est une attaque chimique anisotrope humide d'une tranche de silicium monocristallin dont la structure cristalline est orientée de manière à dégager des plans formant un angle aigu avec la face de ladite tranche lors de l'attaque chimique ;
- les micro-reliefs de la surface de contact ont la forme géométrique d'une pointe ;
- la hauteur des pointes de la surface de contact est comprise entre 50 et 500 micromètres; et
- chaque pointe de la surface de contact à la forme d'une pyramide tronquée à base carrée, la longueur du côté de la base étant comprise entre 150 et 500 micromètres.

D'autres caractéristiques et avantages de l'invention apparaîtront au cours de la description qui va suivre, donnée à titre d'exemple non limitatif, en référence aux dessins annexés dans lesquels :
- la figure 1 est une vue d'ensemble schématique d'un dispositif de mesure d'une caractéristique physique d'un tissu biologique selon l'invention et comprenant un élément de contact ;
- la figure 2 est une vue agrandie de la face de l'élément de contact destinée à venir en appui sur le tissu biologique ; et
- la figure 3 est une coupe longitudinale de l'élément de contact selon la ligne III-III de la figure 2.

Sur les différentes figures, on a conservé les mêmes références pour désigner des éléments identiques ou similaires.

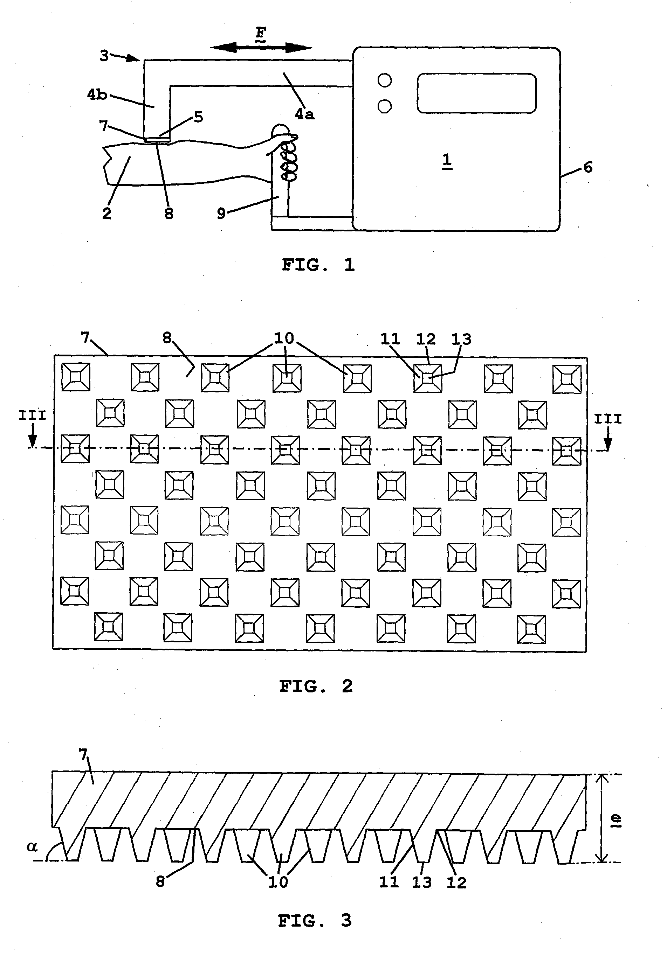

A la figure 1, on a représenté un dispositif de mesure 1 d'au moins une caractéristique physique d'un tissu biologique 2. Dans le mode de réalisation décrit, le dispositif de mesure est plus spécialement destiné à mesurer des caractéristiques physiques de la peau et notamment, dans l'exemple considéré ici, la peau de l'avant-bras d'un être humain.

Le dispositif de mesure 1 comporte un palpeur 3 destiné à venir en appui sur la peau 2 afin d'exercer des sollicitations mécaniques sur celle-ci. Le palpeur 3 est constitué d'un bras 4a terminé par un coude 4b dont l'extrémité 5 vient en appui sur la peau. Toutefois, le palpeur peut avoir une forme différente selon la structure du dispositif de mesure.

Sur l'extrémité 5 du palpeur 3 est fixé un élément de contact 7 présentant une surface de contact 8 destinée à venir en appui sur une portion de la peau 2 à étudier.

Le dispositif de mesure comporte par ailleurs un boîtier 6 à l'intérieur duquel sont logés des moyens permettant de créer des sollicitations sur le palpeur 3 et des moyens de mesure des déplacements ou des efforts résultants des sollicitations.

Il est aussi prévu une poignée 9, solidaire du boîtier 6, afin que le sujet puisse maintenir son avant-bras immobile pendant la mesure.

L'élément de contact 7 est réalisé à partir d'une matière rigide et sa surface de contact 8 présente des micro-reliefs saillants 10 visibles aux figures 2 et 3.

Ainsi, l'élément de contact rigide ne se déforme pas sous l'effet des sollicitations mécaniques, et par conséquent, il n'y a pas de différence de déplacement entre la surface de contact 8 et l'extrémité 5 du palpeur. Ceci permet d'éviter d'introduire une erreur dans la mesure.

De plus, dans le cas de sollicitations dans un plan tangentiel à la surface du tissu biologique pour appliquer des forces de cisaillements, et comme indiqué par la double flèche F, les micro-reliefs 10 permettent d'obtenir un bon accrochage de l'élément de contact 7 sur la peau 2. Ceci' permet d'éviter tout glissement du palpeur par rapport à la peau qui donnerait une mesure erronée.

Bien entendu l'élément de contact doit être fixé de manière parfaitement rigide sur l'extrémité 5 du palpeur 3. Cette fixation peut être réalisée par collage en utilisant une colle appropriée qui n'introduit pas d'élasticité. Mais cette fixation peut aussi être réalisée mécaniquement à l'aide d'une mâchoire serrant l'élément de contact.

La surface de contact 8, ici d'environ 3 mm sur 6 mm, est suffisamment importante pour éviter tout risque d'endommagement de la peau 2. Dans le même but, la hauteur des micro-reliefs est inférieure à 1 millimètre.

De préférence, les micro-reliefs 10 sont répartis uniformément sur toute la surface de contact 8 afin d'obtenir une bonne répartition des forces de cisaillement.

Selon une caractéristique particulièrement avantageuse de l'invention, l'élément de contact 7 est réalisé à partir de silicium. En effet, le silicium présente une bonne rigidité et ne pose pas de problème tant pour son collage sur le palpeur, que pour sa bio-compatibilité.

De plus, ce matériau se prête particulièrement bien à la réalisation des micro-reliefs 10 par un usinage chimique qui permet d'obtenir, à un coût très économique, un grand nombre de micro-reliefs de dimensions précises. Ce type d'usinage consiste essentiellement à protéger certaines zones à l'aide d'un masque, notamment les zones où l'on désire obtenir les micro-reliefs, puis à soumettre le silicium à un produit chimique qui va attaquer les zones non protégées.

Selon un mode de réalisation préféré, l'élément de contact 7 est une plaquette découpée dans une tranche de silicium monocristallin, appelée wafer. On choisi un wafer dont la structure cristalline est orientée de manière à dégager des plans formant un angle aigu α, visible sur la figure 3, avec la surface lors de l'attaque chimique anisotrope humide.

Par exemple, la plaquette est découpé dans un wafer de silicium de coupe [100] en référence aux axes cristallins du silicium utilisé et d'une épaisseur e de 150 à 500 micromètres. Cette plaquette est préalablement protégée par un masque constitué d'une couche de silice obtenue par oxydation. Puis elle est trempée dans une solution qui permet un usinage anisotrope, c'est à dire une solution qui attaque la plaquette à des vitesse différentes selon ses directions cristallines. Des solutions à base d'hydrazine, d'hydroxyde d'ammonium ou d'hydroxyde de potassium permettent d'obtenir une telle attaque et de dégager des plans [111] formant un angle α de 54,74 degrés avec la surface de la plaquette. Ces plans [111] constituent alors les faces latérales 11 des micro-reliefs 10.

En utilisant un masque comportant des ouvertures circulaires disposées en damier, on peut obtenir des micro-reliefs 10 en forme de pyramide tronquée à base carrée 12.

Les micro-reliefs forment ainsi des pointes d'une hauteur comprise entre 50 et 500 micromètres qui offrent un bon accrochage avec la peau et une bonne résistance aux forces de cisaillement car leur base 12 est plus large que leur sommet 13. Dans le mode de réalisation préféré, la longueur du côté de la base 12 est comprise entre 150 et 500 micromètres.

De plus, la forme particulière de pyramide tronquée présente un sommet 13 plat qui évite toute micro-perforation de la peau, tout en offrant un accrochage optimal avec la peau à étudier.

Pour effectuer une mesure de souplesse de la peau à l'aide du dispositif précédemment décrit, on peut exercer une force de traction F sur la peau selon un plan tangentiel à celle-ci et mesurer le déplacement du palpeur 3, les micro-reliefs saillants 10 de l'élément de contact 7 restant bien entendu accrochés au tissu biologique qui subit alors un déplacement relatif fonction de sa souplesse. A partir de ces informations et grâce à l'élément de contact 7, on obtient immédiatement une mesure précise d'une caractéristique physique de la peau.

Bien entendu ce mode de réalisation n'est nullement limitatif. Par exemple, les micro-reliefs 10 peuvent avoir des formes différentes telles que des segments ou des courbes de section tronconique.

## Revendications

1. Dispositif de mesure d'au moins une caractéristique physique d'un tissu biologique (2), comportant un palpeur (3) muni d'un élément de contact (7) présentant une surface de contact (8) destinée à venir en appui sur ledit tissu biologique, des moyens de sollicitation mécanique dudit palpeur et des moyens de mesures des déplacements ou des efforts résultant des sollicitations mécaniques,
**caractérisé en ce que** les moyens de sollicitation mécanique sont adaptés pour solliciter le palpeur (3) dans un plan parallèle à la surface de contact (8) de l'élément de contact,
et **en ce que** l'élément de contact (7) est réalisé à partir d'une matière rigide et la surface de contact (8) dudit élément de contact présente une pluralité de micro-reliefs saillants (10) destinés à venir au contact du tissu.

2. Dispositif de mesure selon la revendication 1, dans lequel la hauteur des micro-reliefs (10) de la surface de contact (8) est inférieure à 1 millimètre.

3. Dispositif de mesure selon la revendication 1 ou 2, dans lequel les micro-reliefs (10) sont uniformément répartis sur toute la surface de contact (8).

4. Dispositif de mesure selon l'une quelconque des revendications 1 à 3, dans lequel les micro-reliefs (10) de la surface de contact (8) sont obtenus par usinage chimique.

5. Dispositif de mesure selon la revendication 4, dans lequel la matière rigide de l'élément de contact (7) est réalisée à partir de silicium.

6. Dispositif de mesure selon la revendication 5, dans lequel l'usinage chimique de l'élément de contact (7) est une attaque chimique anisotrope humide d'une tranche de silicium monocristallin dont la structure cristalline est orientée de manière à dégager des plans (11) formant un angle aigu α avec la face de ladite tranche lors de l'attaque chimique.

7. Dispositif de mesure selon l'une quelconque des revendications 1 à 6, dans lequel les micro-reliefs (10) de la surface de contact (8) ont la forme géométrique d'une pointe.

8. Dispositif de mesure selon la revendication 7, dans lequel la hauteur des pointes (10) de la surface de contact (8) est comprise entre 50 et 500 micromètres.

9. Dispositif de mesure selon la revendication 7 ou 8, dans lequel chaque pointe (10) de la surface de contact (8) à la forme d'une pyramide tronquée à base carrée (12), la longueur du côté de la base (12) étant comprise entre 150 et 500 micromètres.
